# EUROPEAN PATENT APPLICATION

(11) **EP 1 460 135 A1**
(43) Date of publication of application: **22.09.2004**
(21) Application number: 03425778.2
(22) Date of filing: 04.12.2003
(51) Int. Cl.: C12Q 1/46, G01N 33/573

(54) **Acetylcholinesterase method to detect organophosphorate and carbamate insecticides in water, foods and beverages**

(30) Priority: 09.12.2002 IT CH20020017
(71) Applicant: Quality Engineering s.r.l., 65024 Manoppello (PE) (IT)
(72) Inventor: Salfi, Vincenzo, L'Aquila (IT)

(57) **Abstract**

The method of Microplate Acetylcholinesterase Inhibition Assay (from here in then: MAIA) for the test of insecticides in water, food and drink is based on measuring inhibition by insecticide molecule eventually present in the food of reaction rate of acetylcholinesterase (E.C. 3.1.1.7.), from here in then AChE, assayed in microplates.

AChE is preincubated directly with hydroacetonitrilic extract of food matrix in order to enhance the inhibitory effect of insecticide on the same AChE. Subsequently, the AChE is added to its specific reaction substrate, acetylthiocholine iodide, in presence of a suitable chromogenic detector of released thiocholine. After a fixed time the reaction was stopped with a denaturant of the enzymatic protein. It is proceeded, then, to absorbance reading by microplate reader.

The method realizes a biological assay: in fact, the analyte-insecticide is detected and quantified on its specific action harmful *versus* on a physiological event critic of the animal organism: the acetylcholinesterase activity.

## Description

### 1. Progress report

Insecticides determination analytical methods for food matrix:
■ supersensitive chemical methods, quantitative ( HPLC, GC, GC-MS = gas chromatography with mass spectrometry ), official validation methods, of second level, characterized by (i) necessity to know preliminarily the molecular characteristics of the insecticides to be determined, (ii) high cost technology to be involved; ( iii ) high qualification personnel; ( iv ) long times of analysis, globally expensive.
■ Immuno-assay methods, recently worked out, characterized by (i) necessity to know preliminarily molecular characteristics of the insecticides to be determined, (ii) technology to be used of lower cost in comparison with the previous; ( iii ) personnel of elevated qualification; ( iv ) analysis times smaller than the previous.
■ Enzymatic methods including those based on the inhibition of acetylcholinesterase activity, determined by a
■ combined electrode of pH with immobilized enzyme on membrane. Such methods are applied to check up the healthiness of the waters and not of food matrixes that introduce garbage on the membrane.
   The irreversibility of the reaction of inhibition of the immobilized enzyme makes the biosensors liable to a rapid decadence.

### 2. Description

The method of Microplate Acetylcholinesterase Inhibition Assay (from here in then: MAIA) for the test of insecticides in water, food and drink is based on the measure of the inhibition by insecticide molecule eventually present in the food matrix, of the reaction rate of the Acetylcholinesterase (E.C. 3.1.1.7.) (from here in then: AChE), determined in microtitre plates ( from here in then: microplates). It is about of a so called *screening* analytical method, that is of a first level method.
The AChE comes preincubated directly with dried hydroacetonitrilic extract of the food matrix in order to enhance the inhibitory effect on the same AChE by the insecticide. Subsequently, the AChE is set with its specific reaction substrate, Acetylthiocholine iodide ( from here in then: ATCl ) and with an opportune chromogenic reactant for released thiocoline. After a fixed time reaction was stopped with a denaturant of the enzymatic protein. Then it is proceeded to reading absorbance by microplate reader.
The method carries out a truly *biological* assay as the insecticide analyte is detected and quantified on its specific action harmful *versus* a physiological critical event in animal organism: the acetylcholinesterase activity.
The acetylcholinesterase method to detect residuals of organophosphorate and carbamate insecticides in water, food and drink is characterized from the following activities sequence.
1. Homogenization and extraction of the animal or vegetable matrix, if solid, fresh or frozen, analysed for the insecticides. A sample statistically selected of the food in analysis ( 50-100 g ) is added 1:1 with water and ground by a deeping mixer. Subsequently, a share of homogenate is diluted by a right quantity of acetonitrile (in a possible realization of method: ml 2 acetonitrile plus ml 1 homogenate). It is again homogenized in a superhomogenizer (in a possible realization of method: homogenizer type ultra Turrax) for about ten seconds. Then it is centrifuged for about 100,000 gravityxminutes. The post-centrifugational supernatant is the **extract.** The use of the acetonitrile, an excellent organic solvent, a little volatile, mixable with water, optimizes the solubilization of the insecticides from the food matrix with a minimum risk of inhalation for operators.
2. Extract drying by air flux in microplate well, at 30°C temperature, to avoid to deteriorate the insecticide if present. Such an operation performed directly in the well avoids an useless intermediary passage of separate resuspension of extract and addition to the reaction volume.
3. Realization of a reference titre scale (at least three points) of a given insecticide pure for analysis ( in a possible realization of method: CARBARYL, a carbamate, or PARAOXON, an organophosphorate ). the titres are prepared in hydroacetonitrile solution.
4. On a microplate shaker, preincubation for a suitable time ( in a possible realization of method: 30 minutes ), at temperature not superior to 37°C, of the wells containing:
   ■ the analyte ( standard or unknown);
   ■ a neutral pH buffer system that conditions the reaction environment for the better molecular stability of probable insecticides.
   ■ a not ionic surface-active agent (in a possible realization of method: Tween 20), 0.05-0.1%, in order to solubilize dried insecticide;
   ■ the catalytic component made up by fish AChE ( in a possible realization of method: AChE from *Electrophorus electricus*) and by mammal AChE ( in a possible realization of method: AChE from *Bos taurus* ). The use of mixture ( possible with varying percentages from 100% of fish AChE to 100% of mammalian AChE) of enzymes of different origin causes to compound the different sensibility to insecticides of both components and to enlarge, so, the action range of the method;
   ■ the chromogenic detector of thiocholine: 5,5' - Dithio-bis ( 2-nitrobenzoic acid) (from here in then: DTNB). In this phase it is anticipated the addition of the DTNB in order to avoid its premature contact with ATCl, in the absence of enzyme.
   The preincubation increases sensitively anticholinesterasic activity of insecticide molecule;
5. Addition of enzymatic substrate ATCl and incubation for a suitable further period ( in a possible realization of method: 10 minutes);
6. Stop of reaction by inhibiting-denaturing agent (in a possible realization of method: addition 1:3 of 20mM quinidine sulfate in 96° ethanol);
7. Determination of reaction results by absorbance readings by microplate reader (filter photometer) at 405-415nm;
8. Set up of a calibration curve using absorbance net values, in correspondence of concentration points of the footstep 3;
9. Evaluation of samples insecticide titres on terms of reference insecticide equivalents (in a possible realization of method: CARBARYL-equivalents or PARAOXON-equivalents).
   The method involves equipments normally present in an analysis laboratory and requires only general technical competences. The technology of the microplate, as it is known, allows to reduce quantities of kit reagents demolishing cost of used materials for single analysis. The characteristics of lower cost, response speed, stabilization of resultant color after reaction stop, allow to repeat easily the enzyme reaction systems and, then, furnish statistic reliability to the results.
   The enzyme-inhibitor preincubation constitutes a qualifying step of described method.
   The sensibility of described method is at least at a tenth of Maximal Residual Limits at presentation date of current description.

## Claims

1. Acetylcholinesterase method to test organophosphorate and carbamate insecticides in water, food and drink marked by use of microplates for analysis reactions.
The technology of the microplates is very diffused and allows the use of standard equipments and competences for carrying out the method.

2. Acetylcholinesterase method to test organophosphorate and carbamate insecticides in water, food and drink, as for the previous claim, **characterized** from the drying, at 30°C, of the food matrix extract directly in microplate.

3. Acetylcholinesterase method to test organophosphorate and carbamate insecticides in water, food and drink, as for all the previous claims, **characterized by** the preincubation of acetylcholinesterase directly with the dried hydroacetonitrilic extract of food matrix.
The preincubation strengthens the inhibitory effect of probable present insecticide in food matrix on acetylcholinesterase activity.

4. Acetylcholinesterase method to test organophosphorate and carbamate insecticides in water, food and drink, as for all the previous claims, **characterized by** preincubation of acetylcholinesterase directly with hydroacetonitrilic dried extract of food matrix, for a time not less than 30 min and not superior to 90 min.

5. Acetylcholinesterase method to test organophosphorate and carbamate insecticides in water, food and drink, as for all the previous claims, **characterized by** the preincubation of acetylcholinesterase directly with the dried hydroacetonitrilic extract of food matrix, at a temperature not superior to the 37° C.

6. Acetylcholinesterase method to test organophosphorate and carbamate insecticides in water, food and drink, as for all the previous claims, **characterized by** the stop of hydrolysis reaction of acetylthiocoline iodide by a denaturant-inhibitor of acetylcholinesterase.
The stop of reaction after a fixed time allows to carry out the analysis in several microplate wells columns and make comparable readings of the results.

7. Acetylcholinesterase method to test organophosphorate and carbamate insecticides in water, food and drink, as for all the previous claims, **characterized by** the conditioning of reaction environment at neutral pH.
The neutral environment assures, on the one hand, the better molecular stability of the possible insecticides and, from the other, a good reaction rate.

8. Acetylcholinesterase method to test organophosphorate and carbamate insecticides in water, food and drink, as for all the previous claims, **characterized** from the use of a surface-active not ionic agent for solubilization of eventually present insecticide.

9. Acetylcholinesterase method to test organophosphorate and carbamate insecticides in water, food and drink, as for all the previous claims, **characterized** from the use of catalytic component given by a mixture of acetylcholinesterase from fish and mammal.
The mixture allows to exploit the different sensibility to the insecticide molecule of the single components and to enlarge response range of the method.

10. Acetylcholinesterase method to test organophosphorate and carbamate insecticides in water, food and drink, as for all the previous claims, **characterized by** introduction of the chromogenic detector of the thiocoline in the preincubation phase, before introducing reaction substrate.
Avoids the contact direct of the detector with the substrate, before adding the enzyme, to minimize direct effect of detector on hydrolysis of ATCI.
